# EUROPEAN PATENT SPECIFICATION

(11) **EP 0 419 203 B1**
(45) Date of publication and mention of the grant of the patent: **24.08.1994**
(21) Application number: 90310184.8
(22) Date of filing: 18.09.1990
(51) Int. Cl.: A61K 49/02

(54) **Method for rapidly radiolabelling monovalent antibody fragments with technetium**
Methode zur schnellen Radiomarkierung von monovalenten Antikörperfragmenten mit Technetium
Procédé de marquage radioactif rapide de fragments d'anticorps monovalents à l'aide de technétium

(30) Priority: 18.09.1989 US 408241
(43) Date of publication of application: 27.03.1991
(73) Proprietor: Immunomedics, Inc., Morris Plains, New Jersey 07950 (US)
(72) Inventor: Hansen, Hans J., Mystic Island, New Jersey (US); Lentine-Jones, Anastasia, Clinton, New Jersey 08809 (US)
(74) Representative: Perry, Robert Edward

(56) References cited:
- EP-A- 0 005 638
- EP-A- 0 106 608
- EP-A- 0 237 150
- EP-A- 0 336 678
- WO-A-88/07382
- US-E- 32 417

## Description

The present invention relates to a method and kit for directly and rapidly radio-labelling a monovalent antibody fragment with technetium-99m (Tc-99m), using one or more pendant sulfhydryl groups as endogenous ligands, and more particularly to a method and kit for radio-labelling Fab or Fab' antibody fragments to prepare a sterile, Tc-99m-labelled antibody fragment solution which is almost immediately ready for injection into a patient for radioimmunodetection.

EP-A-0336678 discloses direct radio-labelling of antibodies and antibody fragments with various radioisotopes, including Tc-99m and Re-186/188.

EP-A-0237150 and WO-A-8807382 each disclose radio-labelling an antibody or antibody fragment with Tc-99m, but the labelling conditions are not optimised for labelling Fab or Fab' fragments and the disclosed conditions are inconvenient and do not result in quantitative labelling. WO-A-8807382 discloses that, in a preferred embodiment, the radio-labelling can be performed in a single vial by contacting Tc-99m in an oxidised state with a mixture (supplied in lyophilised, frozen or aqueous form) comprised of a sulfhydryl-containing antibody or antibody fragment, a reducing agent and a water-soluble ligand, and incubating the resulting mixture for a time sufficient to allow quantitative radio-labelling of the antibody or antibody fragment.

According to the present invention, a method for producing a sterile, injectable solution of Tc-99m-labelled monovalent antibody fragment, comprises the steps of:
preparing a first sterile solution containing a unit dose for scintigraphic imaging of a monovalent antibody fragment having at least one free sulfhydryl group, stannous chloride in an amount of 10 to 150 »g Sn per mg of antibody fragment, and a 30 to 40-fold molar excess of tartrate over stannous chloride, in 0.04 to 0.06 M acetate buffer containing saline, at a pH of 4.5 to 5.0, wherein the reagents are purged with an inert gas;
maintaining the first sterile solution under an inert gas atmosphere, or making the first sterile solution 0.08 to 1.0 M in sucrose and lyophilising the sugar-containing solution; and
mixing the first sterile solution, or the lyophilisate thereof, with a second sterile solution containing an effective scintigraphic imaging amount of Tc-99m-pertechnetate,
whereby substantially quantitative labelling of the antibody fragment with Tc-99m is effected in about 5 minutes at ambient temperature.

According to a further aspect of the present invention, a sealed, sterile container contains, under an inert gas atmosphere, a first sterile solution as defined above (or a lyophilisate of that solution, made 0.08 to 0.1 M in sucrose), wherein the antibody fragment specifically binds to an antigen produced by or associated with a tumour, an infectious lesion, a microorganism, a parasite, a myocardial infarction, a clot, atherosclerotic plaque, or a normal organ or tissue.

The sterile solution is suitable for immediate injection into a patient for radioimmunodetection.

The present inventors have significantly improved the reagents and conditions for a kit and method for "instant" labeling of monovalent, e.g., Fab or Fab′, antibody fragments containing at least one and preferably a plurality of spatially adjacent stabilized free sulfhydryl groups. Labeling is effected substantially quantitatively at ambient temperature within about 5 minutes of mixing a solution of antibody fragment with pertechnetate, readily available from commercial generators.

Details regarding conventional reagents and procedures are found in EP-A-0 336 678.

It will be understood that the monovalent antibody fragments to be radiolabeled can be fragments which bind to antigens which include but are not limited to antigens produced by or associated with tumors, infectious lesions, microorganisms, parasites, myocardial infarctions, atherosclerotic plaque, or normal organs or tissues. It will also be understood that the term "monovalent antibody fragment" as used herein denotes Fab and Fab′ fragments, normally obtained by cleavage of bivalent fragments or intact immunoglobulin. However, monovalent fragments can also include any fragments retaining the hypervariable, antigen-binding region of an immunoglobulin and having a size similar to or smaller than a Fab′ fragment. This will include genetically engineered and/or recombinant proteins, whether single-chain or multiple-chain, which incorporate an antigen binding site and otherwise function in vivo as targeting vehicles in substantially the same way as natural immunoglobulin fragments.

Fab′ antibody fragments are normally and conveniently made by reductive cleavage of F(ab′)₂ fragments, which themselves are normally made by pepsin digestion of intact immunoglobulin. Cleavage is advantageously effected with thiol reducing agents, e.g., cysteine, mercaptoethanol, dithiothreitol (DTT), glutathione and the like. The cleaved F(ab′)₂ fragment containing at least one free sulfhydryl group will be termed "Fab′-SH" herein. Fab antibody fragments are normally and conveniently made by papain digestion of intact immunoglobulin, preferably in the presence of a thiol reducing agent. Cleaved F(ab)₂ will be termed "Fab-SH" herein.

Reduction of F(ab′)₂ fragments is preferably effected at pH 5.5-7.5, preferably 6.0-7.0, more preferably 6.4-6.8, and most preferably at about pH 6.6, e.g., in citrate, acetate or phosphate buffer, preferably phosphate-buffered saline, and advantageously under an inert gas atmosphere. It is well known that thiol reduction can result in chain separation of the light and heavy chains of the fragment if care is not taken, and the reaction must be carefully controlled to avoid loss of integrity of the fragment.

Cysteine is preferred for such disulfide reductions and other thiols with similar oxidation potentials to cysteine will also be advantageously used. The ratio of disulfide reducing agent to protein is a function of interchain disulfide bond stabilities and must be optimized for each individual case. Cleavage of F(ab′)₂ antibody fragments is advantageously effected with 10-30 mM cysteine, preferably about 20 mM, and a protein concentration of about 10 mg/ml.

Reduction of a F(ab′)₂ fragment with known disulfide bond reducing agents gives after a short time, typically less than one hour, including purification, Fab′ typically having 1-3 free sulfhydryl groups by analysis. Sulfhydryl groups can be introduced into an antibody fragment to improve radiometal binding. Use of Traut's Reagent (iminothiolane) for this purpose is not preferred, whereas use of oligopeptides containing several adjacent sulfhydryl groups is efficacious. In particular, use of metallothionein or, preferably, its C-terminal hexapeptide fragment (hereinafter, "MCTP"), is advantageous.

The Fab-SH or Fab′-SH fragments are advantageously then passed through a short sizing gel column which will trap low molecular weight species, including excess reducing agent. Suitable such sizing gel columns include, e.g., dextrans such as Sephadex® G-25, G-50 (Pharmacia), Fractogel TSK HW55 (EM Science), polyacrylamides such as P-4, P-6 (BioRad), and the like. Cleavage can be monitored by, e.g., size exclusion HPLC, to adjust conditions so that Fab or Fab′ fragments are produced to an optimum extent, while minimizing light-heavy chain cleavage, which is generally less susceptible to disulfide cleavage.

The eluate from the sizing gel column is then stabilized in about 0.03 - 0.07, preferably about 0.05 M acetate buffer, pH about 4.5, made in about 0.1 - 0.3, preferably about 0.15 M saline, and preferably purged with an inert gas, e.g. argon. In general, it is advantageous to work with a concentration of antibody fragment of about 0.5 - 5 mg per ml, preferably about 1 - 3 mg/ml, of solution.

The stabilized Fab-SH or Fab′-SH fragments are next mixed with stannous ion, preferably stannous chloride, and with a stabilizer for the stannous ions. Stannous ion is readily available as its dihydrate, or it can be generated in situ from tin metal, e.g., foil, granules, powder, turnings and the like, by contact with aqueous acid, e.g., HCl. It is usually added in the form of SnCl₂, advantageously in a solution that is also about 0.01 N in HCl, in a ratio of about 10-150, preferably about 123 »g Sn per mg of fragment. Advantageously, the stannous ion solution is prepared by dissolving SnCl·2 H₂O in 6 N HCl and diluting the resultant solution with sterile H₂O that has been purged with argon.

A stabilizing agent for the stannous ion is advantageously present in the solution. It is known that ascorbate can improve specific loading of a chelator with reduced pertechnetate and minimize formation of TcO₂, when the reducing agent is stannous ion. Other polycarboxylic acids, e.g., tartrate, citrate, phthalate, iminodiacetate, ethylenediaminetetraacetic acid (EDTA), diethylenetriaminepentaacetic acid (DTPA) and the like, can also be used. Although polycarboxylic acids are mentioned, by way of illustration, any of a variety of anionic and/or hydroxylic oxygen-containing species could serve this function, e.g., salicylates, acetylacetonates, hydroxyacids, catechols, glycols and other polyols, e.g., glucoheptonate, and the like. Preferred such stabilizers are ascorbate, citrate and tartrate, more preferably tartrate.

While the precise role of such agents is not known, it appears that they chelate stannous ion and may prevent adventitious reactions and/or promote reduction by stabilization of stannic ions, and they may also chelate -- and thereby stabilize -- certain oxidation states of reduced pertechnetate, thereby serving as transchelating agents for the transfer of these technetium ions to the presumably more stable chelation with one or more thiol groups and other nearby ligands on the protein. Such agents will be referred to as "stabilizers" herein. The molar ratio of stabilizer to stannous ion is about 30:1 - 40:1.

A solution of stabilizer, e.g., NaK tartrate, advantageously at a concentration of about 0.1 M, in buffer, preferably sodium acetate, at a pH of about 5.5, is prepared with sterile H₂O purged with argon. One volume of the SnCl₂ solution is mixed with enough of the stabilizer solution to provide a 30 - 40 molar excess, relative to the stannous ion, and the resultant solution is sterile filtered and purged with argon.

The sterile, stabilized SnCl₂ solution is mixed with the sterile Fab′-SH or Fab-SH solution to obtain a final concentration of about 10-150, preferably about 123 »g Sn per mg of fragment. The pH is adjusted, if necessary to about 4.5 - 4.8.

The solution of fragment and stabilized stannous ion is advantageously metered into sterile vials, e.g., at a unit dosage of about 1.25 mg fragment/vial, and the vials are either stoppered, sealed and stored at low temperature, preferably in liquid nitrogen, or lyophilized. In the latter case, the solution is made about 0.09 molar with a sugar such as trehalose or sucrose, preferably sucrose, prior to metering into sterile vials. The material in the vials is then lyophilized, the vacuum is broken with an inert gas, preferably argon, and the vials containing the lyophilizate are stoppered, sealed and stored, optionally in the freezer. The lyophilization conditions are conventional and well known to the ordinary skilled artisan. Both the sealed lyophilizate and the sealed liquid nitrogen stored solution are stable for at least 9 months and retain their capacity to be rapidly and quantitatively labeled with Tc-99m ions upon mixing with pertechnetate.

To label a unit dose of antibody fragment, a vial of liquid nitrogen frozen solution is thawed to room temperature by gentle warming, or a vial of lyphilizate is brought to ambient temperature if necessary, and the seal is broken under inert gas, preferably argon. A sterile saline solution of a suitable imaging quantity of pertechnetate is added to the vial and the contents are mixed. When labeling the foregoing unit dosage quantity of antibody fragment, the amount of pertechnetate is generally about 1 - 100 mCi/mg of antibody fragment, and the time of reaction is about 0.1 - 10 min. With the preferred concentrations of protein and stannous ions noted above, the amount of pertechnetate is preferably about 5 - 20 mCi/mg, and the time of reaction is preferably about 1 - 5 min. This is effectively an "instant" labeling procedure with respect to the prior art processes which generally required 30 minutes to several hours incubation, in some cases at elevated temperatures and/or with additional purification required.

Pertechnetate is generally obtained from a commercially available generator, most commonly in the form of NaTcO₄, normally in saline solution. Other forms of pertechnetate may be used, with appropriate modification of the procedure, as would be suggested by the supplier of a new form of generator or as would be apparent to the ordinary skilled artisan. Pertechnetate is generally used at an activity of about 0.2-20 mCi/ml in saline, e.g., 0.9% ("physiological") sterile saline, optionally buffered at a pH of about 3-7, preferably 3.5-5.5, more preferably about 4.5-5.0. Suitable buffers include, e.g., acetate, tartrate, citrate, phosphate and the like.

The process according to the present invention routinely results in substantantially quantitative incorporation of the label into the antibody fragment in a form which is highly stable to oxidation and resistant to transchelation in saline and serum. When labeled with Tc-99m according to the method of the present invention, 100% incorporation of Tc-99m to Fab′ is seen (within the limits of detection of the analytical monitor) together with >95% retention of immunoreactivity. The radioantibody solutions as prepared above are ready for immediate injection, if done in a properly sterilized, pyrogen-free vial. Also, no blocking of free sulfhydryl groups after technetium binding is necessary for stabilization. Furthermore the immunoreactivity of the labeled fragment is hardly reduced after serum incubation for a day, showing that the conjugates are still completely viable imaging agents out to at least 24 hours.

It will also be apparent to one of ordinary skill that the resultant Tc-99m-radiolabeled antibody fragments are suitable, and in fact particularly convenient and efficacious, in methods of non-invasive scintigraphic imaging of tumors and lesions. In particular, in a method of imaging a tumor, an infectious lesion, a microorganism, a parasite, a myocardial infarction, a clot, atherosclerotic plaque, or a normal organ or tissue, wherein an antibody fragment which specifically binds to an antigen produced by or associated with said tumor, infectious lesion, microorganism, parasite, myocardial infarction, clot, atherosclerotic plaque, or normal organ or tissue, and radiolabeled with a pharmaceutically inert radioisotope capable of external detection, is parenterally injected into a human patient and, after a sufficient time for the radiolabeled antibody or antibody fragment to localize and for non-target background to clear, the site or sites of accretion of the radiolabeled antibody fragment are detected by an external imaging camera, it will be an improvement to use as the radiolabeled antibody fragment a Tc-99m-labeled antibody fragment made according to the method of the present invention. Such imaging methods are well known in the art.

The labeled fragments are also useful for detecting tumors and lesions and defining their boundaries, in intraoperative or endoscopic detection modalities, according to well known methods, e.g., those disclosed in Martin, Jr., et al., U.S. Patent No. 4,782,840, or in Goldenberg, U.S. Patent Application Serial No. 06/943,561. The foregoing scintigraphic, intraoperative and endoscopic methods are all embraced by the term radioimmunodetection.

A kit for use in radiolabeling a monovalent antibody fragment, e.g., an Fab′-SH or Fab-SH fragment, with Tc-99m, using generator-produced pertechnetate, (illustrative of the generic kit as claimed herein, with variations that would be apparent to the ordinary skilled artisan) would typically include about 0.01 - 10 mg, preferably about 1 - 2 mg, per unit dose of an antibody fragment which specifically binds an antigen, e.g., an antigen associated with a tumor, an infectious lesion, a microorganism, a parasite, a myocardial infarction, a clot, atherosclerotic plaque, or a normal organ or tissue, and which contains at least one but preferably a plurality of adjacent free sulfhydryl groups; about 10 - 150 »g per mg of fragment of stannous ions and a 30 - 40 molar excess, relative to the stannous ions, of a stabilizer such as tartrate. The constituents of the kit are provided in a single, sealed sterile vial, in the form of a solution or a lyophilizate, and are mixed just prior to use with about 2 - 100 mCi of Tc-99m pertechnetate per mg of antibody or antibody fragment. Normally, the kit is used and/or provided in combination with one or more auxiliary reagents, buffers, filters, vials, columns and the like for effecting the radiolabeling steps.

The foregoing are merely illustrative and many varients can be envisioned for use with the variations in the process of the invention described hereinabove.

Without further elaboration, it is believed that one skilled in the art can, using the preceding description, utilize the present invention to its fullest extent. In the following examples, all temperatures are set forth uncorrected in degrees Celsius; unless otherwise indicated, all parts and percentages are by weight.

### Example 1

### Preparation of Tc-99m-anti-CEA-Fab′

### A. Labeling Kit

The following solutions are prepared.
(I) A solution of 0.075 M SnCl₂ is prepared by dissolving 3350 mg SnCl·2 H₂O in 1 ml of 6 N HCl and diluting the resultant solution with sterile H₂O that has been purged with argon.
(II) A solution of 0.1 M NaK tartrate in 0.05 M NaAc, at pH 5.5, is prepared with sterile H₂O purged with argon.
(III) One volume of solution I is mixed with 26 volumes of solution II, and the resultant solution is sterile filtered and purged with argon.
(IV) A solution of anti-CEA-Fab′-SH, prepared from a murine monoclonal IgG₁ antibody that specifically binds to carcinoembryonic antigen (CEA) by pepsin cleavage to an F(ab′)₂ fragment, is reduced to Fab′-SH with 20 mM cysteine; excess cysteine is removed by gel filtration, and the Fab′-SH is stabilized (2 mg/ml) at pH 4.5 in 0.05 M NaOAc buffer which is 0.15 M in saline; and the resultant solution is sterile filtered and purged with argon.
(V) Mix solution IV with enough of solution III to obtain a final concentration of 123 »g Sn per mg of Fab′-SH, and adjust the pH to 4.5 - 4.8.

Fill solution V, under argon, into sterile vials (1.25 mg Fab′-SH per vial), stopper, crimp-seal and store vials in liquid nitrogen.

Alternatively, make solution V 0.09 M with sucrose, fill the resultant solution, under argon, into sterile vials (1.25 mg Fab′-SH per vial) and lyophilize. Break the vacuum with argon, stopper the vials containing the lyophilizate and crimp-seal the vials.

### B. Labeled Fragment

Gently warm a vial of liquid nitrogen stored fragment or select a vial of lyophilizate prepared according to part A above. Inject a sterile solution of 10 mCi of sodium pertechnetate in sterile saline from a generator into the vial of Fab′-SH and stabilized stannous ions and mix by gentle agitation. Labeling is quantitative in five minutes, and the resultant solution of Tc-99m-labeled fragment is ready for immediate injection into a patient.

### Example 2

### Tumor Imaging

A sterile solution of a unit dose of Tc-99m-labeled anti-CEA-Fab′ prepared (with liquid nitrogen stored Fab′-SH solution) according to Example 1 is infused intravenously into a patient with a progressively rising CEA titer, the patient having undergone "curative" surgery for a colon carcinoma three years earlier. Scintigraphic imaging 2 h postinjection demonstrates antibody fragment localization in the pelvis at the site of removal of the primary tumor. Subsequent surgery confirms the presence of a 1.0 x 0.5 cm carcinoma that is successfully removed.

### Example 3

### Tumor Imaging

A sterile solution of a unit dose of Tc-99m-labeled anti-CEA-Fab′ prepared (from lyophilizate) according to Example 1 is infused intravenously into a patient with a 3 x 2 cm rectal polyp that has been proven by biopsy to be malignant. Imaging 2 h postinjection demonstrates localized antibody fragment in the primary tumor, the right lobe of the liver and in the lower lobe of the left lung. Needle biopsy confirms the presence of tumor in both the liver and the lung. The original plan to perform surgery and adjuvant radiation therapy is abandoned and palliative chemotherapy is instituted.

The preceding examples can be repeated with similar success by substituting the generically or specifically described reactants and/or operating conditions of this invention for those used in the preceding examples.

## Claims

1. A method for producing a sterile, injectable solution of Tc-99m-labelled monovalent antibody fragment, which comprises the steps of:
preparing a first sterile solution containing a unit dose for scintigraphic imaging of a monovalent antibody fragment having at least one free sulfhydryl group, stannous chloride in an amount of 10 to 150 »g Sn per mg of antibody fragment, and a 30 to 40-fold molar excess of tartrate over stannous chloride, in 0.04 to 0.06 M acetate buffer containing saline, at a pH of 4.5 to 5.0, wherein the reagents are purged with an inert gas;
maintaining the first sterile solution under an inert gas atmosphere, or making the first sterile solution 0.08 to 1.0 M in sucrose and lyophilising the sugar-containing solution; and
mixing the first sterile solution, or the lyophilisate thereof, with a second sterile solution containing an effective scintigraphic imaging amount of Tc-99m-pertechnetate,
whereby substantially quantitative labelling of the antibody fragment with Tc-99m is effected in about 5 minutes at ambient temperature.

2. The method of claim 1, wherein the monovalent antibody fragment is a Fab-SH or Fab'-SH fragment.

3. The method of claim 1, wherein the antibody fragment specifically binds a tumour marker.

4. The method of claim 1, wherein the antibody fragment specifically binds an antigen associated with an infectious lesion, a microorganism or a parasite.

5. The method of claim 1, wherein the antibody fragment specifically binds an antigen associated with a myocardial infarction, a clot or atherosclerotic plaque.

6. The method of claim 1, wherein the antibody fragment specifically binds an antigen associated with a normal organ or tissue.

7. The method of any preceding claim, wherein the inert gas is argon.

8. A sealed, sterile container containing, under an inert gas atmosphere, a first sterile solution as defined in claim 1, wherein the antibody fragment specifically binds to an antigen produced by or associated with a tumour, an infectious lesion, a microorganism, a parasite, a myocardial infarction, a clot, atherosclerotic plaque, or a normal organ or tissue.

9. A sealed, sterile container containing a lyophilisate of a first sterile solution as defined in claim 1, made 0.08 to 0.1 M in sucrose, wherein the antibody fragment is as defined in claim 8.

## Patentansprüche

1. Verfahren zur Herstellung einer sterilen, injizierbaren Lösung von mit Tc-99m-markiertem, einwertigem Antikörperfragment, umfassend folgende Stufen:
Herstellen einer ersten sterilen Lösung mit einem Gehalt an einer Einheitsdosis für die szintigraphische Abbildung eines einwertigen Antikörperfragments mit mindestens einer freien Sulfhydrylgruppe, Zinn(II)-chlorid in einer Menge von 10 bis 150 »g Sn pro mg Antikörperfragment und einem 30- bis 40-fachen molaren Überschuß eines Tartrats gegenüber dem Zinn(II)-chlorid, in 0,04 bis 0,06 m Acetatpuffer mit einem Gehalt an Kochsalz und einem pH-Wert von 4,5 bis 5,0, wobei die Reagenzien mit einem Inertgas gespült werden;
Belassen der ersten sterilen Lösung unter einer inerten Gasatmosphäre oder Einstellen der ersten sterilen Lösung auf einen Saccharosegehalt von 0,08 bis 1,0 m und Lyophilisieren der zuckerhaltigen Lösung; und
Vermischen der ersten sterilen Lösung oder des Lyophilisats davon mit einer zweiten sterilen Lösung, die eine für die szintigraphische Abbildung wirksame Menge an Tc-99m-Pertechnetat enthält,
wobei eine im wesentlichen quantitative Markierung des Antikörperfragments mit Tc-99m in etwa 5 Minuten bei Umgebungstemperatur erreicht wird.

2. Verfahren nach Anspruch 1, wobei es sich beim einwertigen Antikörperfragment um Fab-SH- oder Fab'-SH-Fragment handelt.

3. Verfahren nach Anspruch 1, wobei das Antikörperfragment spezifisch an einen Tumormarker bindet.

4. Verfahren nach Anspruch 1, wobei das Antikörperfragment spezifisch an ein Antigen, das mit einer infektiösen Läsion, einem Mikroorganismus oder einem Parasiten assoziiert ist, bindet.

5. Verfahren nach Anspruch 1, wobei das Antikörperfragment spezifisch an ein Antigen, das mit einem Myokardinfarkt, einem Gerinnsel oder einer atherosklerotischen Plaque assoziiert ist, bindet.

6. Verfahren nach Anspruch 1, wobei das Antikörperfragment spezifisch an ein Antigen, das mit einem normalen Organ oder Gewebe assoziiert ist, bindet.

7. Verfahren nach einem der vorstehenden Ansprüche, wobei es sich beim Inertgas um Argon handelt.

8. Verschlossener, steriler Behälter, enthaltend unter Inertgasatmosphäre eine erste sterile Lösung gemäß der Definition in Anspruch 1, wobei das Antikörperfragment spezifisch an ein Antigen bindet, das von einem Tumor, einer infektiösen Läsion, einem Mikroorganismus, einem Parasiten, einem Myokardinfarkt, einem Gerinnsel, einer atherosklerotischen Plaque oder einem normalen Organ oder Gewebe gebildet worden oder damit assoziiert ist.

9. Verschlossener, steriler Behälter, enthaltend ein Lyophilisat einer ersten sterilen Lösung gemäß der Definition in Anspruch 1, die auf eine Saccharosekonzentration von 0,08 bis 0,1 m gebracht worden ist, wobei das Antikörperfragment der Definition in Anspruch 8 entspricht.

## Revendications

1. Méthode de production d'une solution stérile injectable d'un fragment d'anticorps monovalent marqué au Tc-99m, qui comprend les étapes selon lesquelles:
on prépare une première solution stérile contenant une dose unitaire pour la visualisation en scintigraphie d'un fragment d'anticorps monovalent ayant au moins un groupe sulfhydryle libre, du chlorure stanneux en une quantité correspondant à 10 à 150 »g de Sn par mg de fragment d'anticorps, et un excès 30 à 40 fois molaire de tartrate par rapport au chlorure stanneux, dans une solution salée contenant un tampon acétate 0,04 à 0,06 M, à un pH de 4,5 à 5,0, les réactifs étant purgés avec un gaz inerte;
on maintient la première solution stérile sous une atmosphère de gaz inerte, ou on introduit du sucrose dans la première solution stérile à une concentration de 0,08 à 1,0 M et on lyophilise la solution contenant le sucre; et
on mélange la première solution stérile ou son produit lyophilisé avec une seconde solution stérile contenant une quantité efficace pour la visualisation en scintigraphie de pertechnétate de Tc-99m,
grâce à quoi le marquage essentiellement quantitatif du fragment d'anticorps avec le Tc-99m s'effectue en près de 5 minutes à la température ambiante.

2. Méthode selon la revendication 1, dans laquelle le fragment d'anticorps monovalent est un fragment Fab-SH ou Fab'-SH.

3. Méthode selon la revendication 1, dans laquelle le fragment d'anticorps fixe de façon spécifique un marqueur tumoral.

4. Méthode selon la revendication 1, dans laquelle le fragment d'anticorps fixe de façon spécifique un antigène associé à une lésion infectieuse, un microorganisme ou un parasite.

5. Méthode selon la revendication 1, dans laquelle le fragment d'anticorps fixe de façon spécifique un antigène associé à un infarctus du myocarde, un caillot ou une plaque d'athérosclérose.

6. Méthode selon la revendication 1, dans laquelle le fragment d'anticorps fixe de façon spécifique un antigène associé à un organe ou tissu normal.

7. Méthode selon l'une quelconque des revendications précédentes, dans laquelle le gaz inerte est l'argon.

8. Récipient stérile scellé contenant, sous atmosphère de gaz inerte, une première solution stérile telle que définie dans la revendication 1, dans laquelle le fragment d'anticorps fixe de façon spécifique un antigène produit par, ou associé à, une tumeur, une lésion infectieuse, un microorganisme, un parasite, un infarctus du myocarde, un caillot, une plaque d'athérosclérose ou un organe ou tissu normal.

9. Récipient stérile scellé contenant un produit lyophilisé d'une première solution stérile telle que définie dans la revendication 1, contenant du sucrose à une concentration de 0,08 à 0,1 M, dans laquelle le fragment d'anticorps est tel que défini dans la revendication 8.
